# EUROPEAN PATENT SPECIFICATION

(11) **EP 3 634 515 B1**
(45) Date of publication and mention of the grant of the patent: **23.02.2022**
(21) Application number: 18726003.9
(22) Date of filing: 02.05.2018
(51) Int. Cl.: A61M 1/00, A61F 13/00, A61F 13/02

(54) **TISSUE INTERFACE INCORPORATING MULTIPLE LAYERS**
GEWEBESCHNITTSTELLE MIT MEHREREN SCHICHTEN
INTERFACE TISSULAIRE COMPRENANT DE MULTIPLES COUCHES

(30) Priority: 07.06.2017 US 201762516451 P
(43) Date of publication of application: 15.04.2020
(73) Proprietor: 3M Innovative Properties Company, St. Paul, MN 55133-3427 (US)
(72) Inventor: LOCKE, Christopher, Brian, Bournemouth BH9 3SD (GB); ROBINSON, Timothy, Mark, Shillingstone DT11 0TG (GB)
(74) Representative: Simmons & Simmons
(86) International application number: PCT/US2018/030568
(87) International publication number: WO 2018/226328

(56) References cited:
- WO-A1-2015/173546
- WO-A1-2016/182977
- WO-A1-2018/081217
- US-A1- 2012 143 157
- US-A1- 2013 041 337
- US-A1- 2015 245 949
- US-A1- 2016 022 885
- US-A1- 2017 079 846
- US-A1- 2017 151 095

## Description

### TECHNICAL FIELD

The invention set forth in the appended claims relates generally to tissue treatment systems and more particularly, but without limitation, to a tissue interface having multiple detachable layers for use in conjunction with reduced pressure wound therapy.

### BACKGROUND

Clinical studies and practice have shown that reducing pressure in proximity to a tissue site may augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "reduced-pressure therapy," but is also known by other names, including "negative-pressure wound therapy," "negative-pressure therapy," "vacuum therapy," "vacuum-assisted closure," and "topical negative-pressure," for example. Reduced-pressure therapy may provide a number of benefits, including migration of epithelial and subcutaneous tissues, improved blood flow, and micro-deformation of tissue at a wound site. Together, these benefits may increase development of granulation tissue and reduce healing times.

Tissue treatment systems may use a tissue interface (such as a wound manifold or a wound filler) that facilitates various degrees of tissue ingrowth when disposed at a tissue site, whether on or within a wound at a tissue site, that may cause pain or discomfort to a patient and in some cases leave remnants of the tissue interface in the wound when the tissue interface is removed from the tissue site. Accordingly, improvements to the tissue interface are desirable.

WO 2015/173546 A1 concerns a dressing for surgical drain. US 2012/143157 A1 concerns a multi-component dressing for wound treatment on the human or animal body with application of reduced pressure. US 2013/041337 A1 concerns systems and methods for protecting incisions. US 2017/079846 A1 concerns a hybrid silicone and acrylic adhesive cover for use with wound treatment. US 2016/022885 A1 concerns a negative pressure wound closure device and systems and methods of use in treatment wounds with negative pressure. US 2017/151095 A1 concerns a flexible, adherent, and non-polyurethane film wound drape cover. US 2015/245949 A1 concerns a hybrid drape having a gel-coated perforated mesh. WO 2016/182977 A1 concerns a low acuity dressing with integral pump. WO 2018/081217 A1 (pursuant to Article 54(3) EPC) concerns a hybrid drape and method of manufacturing a hybrid drape.

While the clinical benefits of reduced-pressure therapy and tissue interfaces are widely known, improvements to therapy systems, components, and processes may benefit healthcare providers and patients.

### BRIEF SUMMARY

There is provided a tissue interface configured to be positioned at a tissue site, the tissue interface comprising: a first tissue interface layer having a surface adapted to promote granulation of tissue when placed on tissue under negative pressure; a second tissue interface layer; and a first bonding layer positioned between the first tissue interface layer and the second tissue interface layer and having a bonding strength for coupling the first tissue interface layer to the second tissue interface layer, wherein the bonding strength is sufficiently weak for allowing the first tissue interface layer to be separated from the second tissue interface layer when a force greater than the bonding strength is applied to pull the first tissue interface layer away from the second tissue interface layer, wherein the first bonding layer comprises an adhesive, the bonding strength of the first bonding layer is between 1.0N per mm² and 1.5N per mm² when cured.

A selection of optional features is set out in the dependent claims

### BRIEF DESCRIPTION OF THE DRAWINGS

Figure 1 is a simplified functional block diagram of an example embodiment of a therapy system for providing reduced-pressure therapy comprising a tissue interface;
Figure 2 is a perspective view of a first example embodiment of a tissue interface for use in the therapy system of Figure 1;
Figure 3 is a perspective view of a second example embodiment of a tissue interface for use in the therapy system of Figure 1;
Figure 4 is a perspective view of a third example embodiment of a tissue interface for use in the therapy system of Figure 1;
Figure 5 is a perspective view of an example embodiment of a tissue interface including a tissue interface layer and a bonding layer positioned over an entire surface of the tissue interface layer;
Figure 6 is a perspective view of an example embodiment of a tissue interface including a tissue interface layer and a bonding layer having a first pattern positioned on a surface of the tissue interface layer;
Figure 7 is a perspective view of an example embodiment of a tissue interface including a tissue interface layer and a bonding layer having a second pattern positioned on a surface of the tissue interface layer;
Figure 8 is a perspective view of an example embodiment of a tissue interface including a tissue interface layer and a bonding layer having spot bonding segments positioned on a surface of the tissue interface layer;
Figure 9 is a perspective view of an example embodiment of a tissue interface including a bed of nails structure for use in the therapy system of Figure 1;
Figure 10 is a perspective view of an example embodiment of a tissue interface including a bonding layer having a hook and loop structure;
Figure 11 is a perspective view of an example embodiment of a tissue interface including a bonding layer having a dome and spike structure;
Figure 12 is a perspective view of an example embodiment of a tissue interface including a cylindrical structure for use in the therapy system of Figure 1; and
Figure 13 is perspective view of an example embodiment of a tissue interface including a coiled structure for use in the therapy system of Figure 1.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

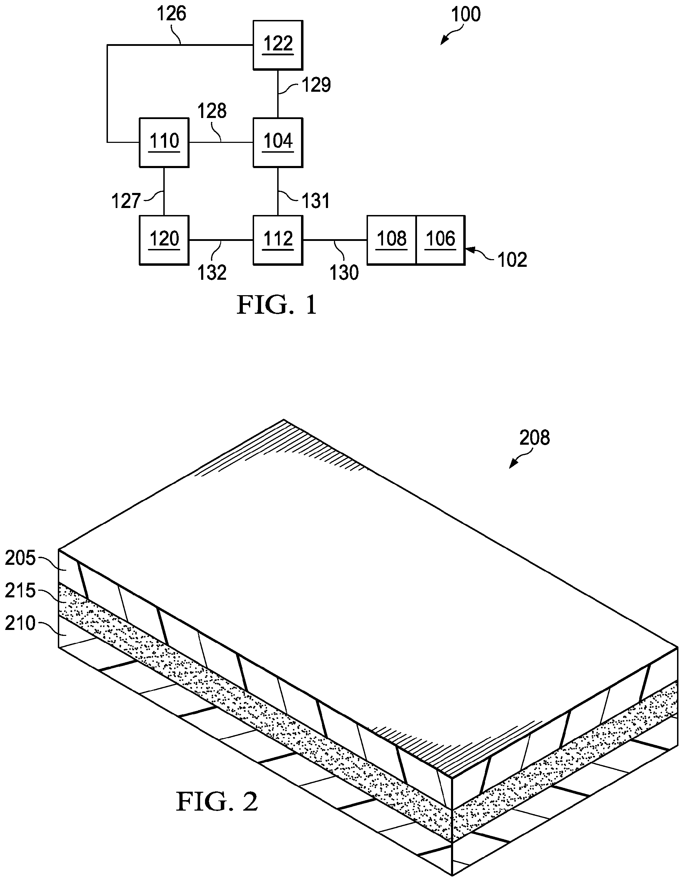

Figure 1 is a simplified functional block diagram of an example embodiment of a therapy system 100 for providing reduced-pressure therapy comprising a tissue interface. The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, reduced pressure may be applied to a tissue site to grow additional tissue that may be harvested and transplanted.

The therapy system 100 may include reduced-pressure supply, and may include or be configured to be coupled to a distribution component or tubing, such as a dressing. In general, a distribution component may refer to any complementary or ancillary component configured to be fluidly coupled to a reduced-pressure supply in a fluid path between a reduced-pressure supply and a tissue site. A distribution component is preferably detachable, and may be disposable, reusable, or recyclable. For example, a dressing 102 may be fluidly coupled to a reduced-pressure source 104, as illustrated in Figure 1. A dressing may include a cover, a tissue interface, or both in some embodiments. The dressing 102, for example, may include a cover 106 and a tissue interface 108. A regulator or a controller, such as a controller 110, may also be coupled to the reduced-pressure source 104.

In some embodiments, a dressing interface may facilitate coupling the reduced-pressure source 104 to the dressing 102. For example, such a dressing interface may be a T.R.A.C.^{®} Pad or Sensa T.R.A.C.^{®} Pad available from KCI of San Antonio, Texas. The therapy system 100 may optionally include a fluid container, such as a container 112, coupled to the dressing 102 and to the reduced-pressure source 104.

Additionally, the therapy system 100 may include sensors to measure operating parameters and provide feedback signals to the controller 110 indicative of the operating parameters. As illustrated in Figure 1, for example, the therapy system 100 may include at least one of a pressure sensor 120 or an electric sensor 122 coupled to the controller 110 via electric conductors 126 and 127, respectively. The pressure sensor 120 may also be coupled or configured to be fluidly coupled via a distribution component such as, for example, fluid conduits 131 and 132 and to the reduced-pressure source 104. For example, as shown in Figure 1, the electric conductor 126 provides electric communication between the electric sensor 122 and the controller 110. The electric conductor 127 provides electric communication between the pressure sensor 120 and the controller 110. The electric conductor 128 provides electric communication between the controller 110 and the reduced-pressure source 104. The electric conductor 129 provides electric communication between the electric sensor 122 and the reduced-pressure source 104.

Components may be fluidly coupled to each other to provide a path for transferring fluids (such as at least one of liquid or gas) between the components. Components may be fluidly coupled through a fluid conductor, such as a tube. For example fluid conductor 130 provides fluid communication between the dressing 102 and the container 112; fluid conductor 131 provides fluid communication between the reduced-pressure source 104 and the container 112; and fluid conductor 132 provides fluid communication between the pressure sensor 120 and the container 112. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors 124 may be molded into or otherwise integrally combined with other components. Coupling may also include mechanical, thermal, electrical, or chemical coupling (such as a chemical bond) in some contexts. For example, a tube may mechanically and fluidly couple the dressing 102 to the container 112 in some embodiments.

In general, components of the therapy system 100 may be coupled directly or indirectly. For example, the reduced-pressure source 104 may be directly coupled to the controller 110, and may be indirectly coupled to the dressing 102 through the container 112. The fluid mechanics of using a reduced-pressure source to reduce pressure in another component or location, such as within a sealed therapeutic environment, can be mathematically complex. However, the basic principles of fluid mechanics applicable to reduced-pressure therapy are generally well-known to those skilled in the art, and the process of reducing pressure may be described illustratively herein as "delivering," "distributing," or "generating" reduced pressure, for example.

In general, exudates and other fluids flow toward lower pressure along a fluid path. Thus, the term "downstream" typically implies something in a fluid path relatively closer to a source of reduced pressure or further away from a source of positive pressure. Conversely, the term "upstream" implies something relatively further away from a source of reduced pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a reduced-pressure source) and this descriptive convention should not be construed as a limiting convention.

"Reduced pressure" or "negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in reduced pressure typically refer to a decrease in absolute pressure, while decreases in reduced pressure typically refer to an increase in absolute pressure. While the amount and nature of reduced pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -75 mm Hg (-9.9 kPa) and -300 mm Hg (-39.9 kPa).

A reduced-pressure supply, such as the reduced-pressure source 104, may be a reservoir of air at a reduced pressure, or may be a manual or electrically-powered device that can reduce the pressure in a sealed volume, such as a vacuum pump, a suction pump, a wall suction port available at many healthcare facilities, or a micro-pump, for example. A reduced-pressure supply may be housed within or used in conjunction with other components, such as sensors, processing units, alarm indicators, memory, databases, software, display devices, or user interfaces that further facilitate therapy. For example, in some embodiments, the reduced-pressure source 104 may be combined with the controller 110 and other components into a therapy unit. A reduced-pressure supply may also have one or more supply ports configured to facilitate coupling and de-coupling the reduced-pressure supply to one or more distribution components.

Sensors, such as the pressure sensor 120 or the electric sensor 122, are generally known in the art as any apparatus operable to detect or measure a physical phenomenon or property, and generally provide a signal indicative of the phenomenon or property that is detected or measured. For example, the pressure sensor 120 and the electric sensor 122 may be configured to measure one or more operating parameters of the therapy system 100. In some embodiments, the pressure sensor 120 may be a transducer configured to measure pressure in a pneumatic pathway and convert the measurement to a signal indicative of the pressure measured. In some embodiments, for example, the pressure sensor 120 may be a piezoresistive strain gauge. The electric sensor 122 may optionally measure operating parameters of the reduced-pressure source 104, such as the voltage or current, in some embodiments. Preferably, the signals from the pressure sensor 120 and the electric sensor 122 are suitable as an input signal to the controller 110, but some signal conditioning may be appropriate in some embodiments. For example, the signal may need to be filtered or amplified before it can be processed by the controller 110. Typically, the signal is an electrical signal, but may be represented in other forms, such as an optical signal.

The container 112 may be representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with reduced-pressure therapy.

In some embodiments, the cover 106 may provide a bacterial barrier and protection from physical trauma. The cover 106 may also be constructed from a material that can reduce evaporative losses and provide a fluid seal between two components or two environments, such as between a therapeutic environment and a local external environment. The cover 106 may be, for example, an elastomeric film or membrane that can provide a seal adequate to maintain a reduced pressure at a tissue site for a given reduced-pressure source. The cover 106 may have a high moisture-vapor transmission rate (MVTR) in some applications. For example, the MVTR may be at least 300 g/m^2 per twenty-four hours in some embodiments. In some example embodiments, the cover 106 may be a polymer drape, such as a polyurethane film, that is permeable to water vapor but impermeable to liquid. Such drapes typically have a thickness in the range of 25-50 µm (microns). For permeable materials, the permeability generally should be low enough that a desired reduced pressure may be maintained.

An attachment device may be used to attach the cover 106 to an attachment surface, such as undamaged epidermis, a gasket, or another cover. The attachment device may take many forms. For example, an attachment device may be a medically-acceptable, pressure-sensitive adhesive that extends about a periphery, a portion, or an entire sealing member. In some embodiments, for example, some or all of the cover 106 may be coated with an acrylic adhesive having a coating weight between 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The tissue interface 108 may be configured to contact a tissue site. The tissue interface 108 may be partially or fully in contact with the tissue site. If the tissue site is a wound, for example, the tissue interface 108 may partially or completely fill the wound, or may be placed over the wound. The tissue interface 108 may take many forms, and may have many sizes, shapes, or thicknesses depending on a variety of factors, such as the type of treatment being implemented or the nature and size of a tissue site. For example, the size and shape of the tissue interface 108 may be adapted to the contours of deep and irregular shaped tissue sites. Moreover, any or all of the surfaces of the tissue interface 108 may have projections or an uneven, course, or jagged profile that can induce strains and stresses on a tissue site, which can promote granulation at the tissue site.

In some embodiments, the tissue interface 108 may be a manifold. A "manifold" in this context generally includes any substance or structure providing a plurality of pathways adapted to collect or distribute fluid across a tissue site under pressure. For example, a manifold may be adapted to receive reduced pressure from a source and distribute reduced pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site. The tissue interface 108 may also be a wound filler or spacer material that includes a substance or structure that does not provide a plurality of pathways adapted to collect or distribute fluid across a tissue site under pressure. The tissue interface 108 may include both a manifold and a spacer material in some embodiments to both distribute fluid and fill the space between the cover 106 and a tissue site. The tissue interface 108 may comprise one or more materials that have a variety of shapes including, for example, materials having a sheet-like shape or thicker materials having more of a three-dimensional shape. The tissue interface 108 may have a variety of geometric shapes such as circular shapes or rectangular shapes, or a variety of irregular shapes.

In operation, referring to at least Figure 1, the tissue interface 108 may be sized and shaped to be adapted to the contours of a deep and irregular shaped tissue site as described above. The tissue interface 108 may then be placed within, over, on, or otherwise proximate to a tissue site. After positioning the tissue interface 108, the tissue interface 108 may be sized and shaped again to better fit within, over, on, or otherwise proximate to a tissue site. For example, the perimeter of the tissue interface 108 may be trimmed to fit within the contours of a tissue site. The cover 106 may be placed over the tissue interface 108 and sealed to an attachment surface that may be near, around, or adjacent to the tissue site. For example, the cover 106 may be sealed to undamaged epidermis peripheral to a tissue site. Thus, the dressing 102 may provide a sealed therapeutic environment proximate to a tissue site, substantially isolated from the external environment, and the reduced-pressure source 104 may reduce the pressure in the sealed therapeutic environment. Reduced pressure applied across the tissue site through the tissue interface 108 in the sealed therapeutic environment may induce macrostrain and microstrain in the tissue site, as well as remove exudates and other fluids from the tissue site, which can be collected in container 112.

In some example embodiments, the tissue interface 108 may comprise two or more tissue interface layers bonded together lightly with one or more bonding layers. In operation, the height of the tissue interface 108 may be adjusted by using multiple tissue interface layers to accommodate the depth of a tissue site. In one example embodiment, the tissue interface layers may have a thickness that is less than about 10 mm. In another example embodiment, the tissue interface layers may have a thickness between about 1.0 mm and about 10 mm. The interface layers are bonded together lightly such that the bonding layer is sufficiently strong to hold together a first interface layer and a second interface layer, but weak enough to allow the first tissue interface layer to be separated or detached from the second tissue interface layer when pulled apart by a user. This allows a user to add or remove any number of tissue interface layers to adjust the height of the tissue interface 108 in order to accommodate the depth of a tissue site. The tissue interface layers may have different thicknesses and a variety of shapes. The tissue interface layers may comprise one or more materials that have a variety of shapes including, for example, materials having a sheet-like shape or thicker materials having more of a three-dimensional shape. The tissue interface layers may have a variety of geometric shapes such as circular shapes or rectangular shapes, or a variety of irregular shapes.

The tissue interface layers may comprise fabric materials having characteristics such as, for example, non-shedding fabrics, elastic fabrics, hydrophobic fabrics, and hydrophilic fabrics. An example embodiment of a three-dimensional fabric may include 3MESH^{™} available from MULLER^{™}. The fabrics may comprise a framework or structure in the form of a mesh, a mat, or other forms known by those having ordinary skill in the art. The fabrics may serve a variety of functions such as, for example, a manifold fabric as described above, a spacer fabric, or a combination of several functions. An example embodiment of a spacer fabric may include HEATHCOAT^{™}, and XD SPACER^{™} available from BALTEX^{™}. The tissue interface layers may comprise other materials such as, for example, composite materials, woven gauzes, polymeric materials (such as polymeric films, non-foamed materials, polyolefin bonded fiber materials, polymer extruded gauzes, welded polymer meshes, and vacuum formed polymer bubble wrap), non-woven materials, bed of nails molded structures, a patterned grid material (such as those manufactured by Sercol Industrial Fabrics), elastic materials, or any other material known by those having ordinary skill in the art. Example embodiments of polymeric films may include thermoplastic elastomers (TPE), thermoplastic vulcanizates (TPV), thermoplastic polyurethane (TPU), polyether block amide (PEBAX), and any other material known by those having ordinary skill in the art. Working examples of polymeric films may include DELNET^{®} Apertured Films and STRATEX^{®} Engineered Composites from DelStar Technologies, Inc. of Middletown, Delaware. Working examples of non-woven materials such as polyolefin fiber materials include those polyolefin bonded fiber materials available from ESSENTRA^{®}.

In some embodiments, the tissue interface layers may include an elastic material including a material that is elastic in compression or elastic in expansion. For example, a tissue interface layer may be compressed or stretched in a plurality of different directions and return to its initial shape and size when compression or stretching ceases. In some embodiments, the tissue interface layer may be expanded to form a 3D structure such as a scaffold. The tissue interface layers may serve as a scaffold to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials. In various embodiments, the tissue interface layers may be constructed from bioresorbable materials. When the tissue interface layers are constructed from bioresorbable materials, if a portion of a tissue interface layer is left within the tissue site after removal of the tissue interface 108, the portion of the tissue interface layer may not cause infection or irritation at the tissue site. Suitable bioresorbable materials may include, without limitation, a polymeric blend of polylactic acid (PLA) and polyglycolic acid (PGA). A polymeric blend may also include without limitation at least one of polycarbonates, polyfumarates, or capralactones.

In at least some embodiments, the tissue interface layers may be treated. For example, a tissue interface layer may be treated or coated with a hydrophilic treatment or an elastic film. Hydrophilic treatments may include a coating or a hydrophilic adhesive having a pattern. Elastic films may include a thermoplastic elastomer (TPE), a thermoplastic vulcanizates (TPV), thermoplastic polyurethane (TPU), or a polyether block amide

(PEBAX). The tissue interface layers may be treated with a hydrophilic treatment or an elastic film to provide at least one of fluid transport, wicking, or absorbent properties. For example, a tissue interface layer of the tissue interface 108 treated with a hydrophilic treatment or an elastic film may allow the tissue interface 108 to wick fluid away from a tissue site, while continuing to distribute reduced pressure to the tissue site.

The tissue interface layer may comprise a sheet-like material that is perforated and/or textured. The tissue interface layer may be perforated to include one or more fenestrations or perforations extending through any of the embodiments of the tissue interface layer described above. The perforations may communicate fluid through the tissue interface layer to enhance wound therapy including negative pressure wound therapy. The tissue interface layer may also include a surface having one or more indentations or hollows on the surface that do not extend through the tissue interface layer. Such indentations or hollows may have a variety of shapes such as, for example, one or more dimples or channels.

As indicated above, the tissue interface layer may have a generally sheet-like shape which may include a textured surface on at least one side of the sheet or a portion of one side of the sheet. In one example embodiment, the textured surface of the tissue interface layer may include a pattern or arrangement of particles or constituent parts of the tissue interface layer. In yet another example embodiment, the textured surface of the tissue interface layer may include any flexible or rigid protrusions extending from one side of the sheet. Such patterns or protrusions may have a variety of shapes such as, for example, pyramids, cylinders, ribs, or other non-symmetrical shapes. The protrusions may be arranged in regular or irregular patterns, and the patterns themselves may be irregular and/or non-symmetrically formed on the surface of the tissue interface layer. The protrusions of the textured surface may be formed with either a course or fine grain surface. The textured surface may be formed by embossing methods that cover the surface of the tissue interface with patterns of protrusions raised above the surface of the tissue interface layer.

In some embodiments of the tissue interface layer having perforations, the perforations may be formed by punching holes through the tissue interface layer, perforating the tissue interface layer, cutting holes in the tissue interface layer, or any number of different methods. The perforations also may be formed by vacuum forming methods. The perforations may be formed into one or more shapes including, for example, an elliptical shape including a circle, a polygon, an oval, or a simple slit through the material. Perforations having a generally elliptical shape may have diameters between about 300 microns and about 1000 microns. The perforations may be stretched in different directions to change the shape of the perforations. Stretching the tissue interface layer in one or more directions to form different shapes and sizes facilitates fluid communication through the tissue interface layer. The perforations may be positioned randomly throughout the tissue interface layer. In at least some embodiments, the perforations may be positioned so that one or more perforations are more concentrated in one region of the tissue interface layer while other perforations are less concentrated in other regions of the tissue interface layer. In some embodiments of the tissue interface layer, the perforations may be formed through the tissue interface layer in combination with one or more of the other features described herein.

As indicated above, two or more tissue interface layers may be bonded together lightly so that the bonding layer is sufficiently strong to hold together a first interface layer and a second interface layer, but weak enough to allow the first tissue interface layer to be separated or detached from the second tissue interface layer when pulled apart by a user. The bonding layer may be, for example, an adhesive that is biocompatible and does not alter the performance of the tissue interface 108. Example embodiments of adhesives may include a silicone gel (such as a silicone gel that cures at room temperature), a polyurethane (PU) gel, an acrylic gel, a hydrogel, a hydrocolloid, a rubber-based adhesive (such as butyl rubber), a pressure-sensitive adhesive, cool-melt or hot-melt adhesives (such as a PU acrylic, ethylene-vinyl acetate (EVA), or other chemistries), any other adhesives known by those having ordinary skill in the art, or a combination of any of the foregoing adhesives.

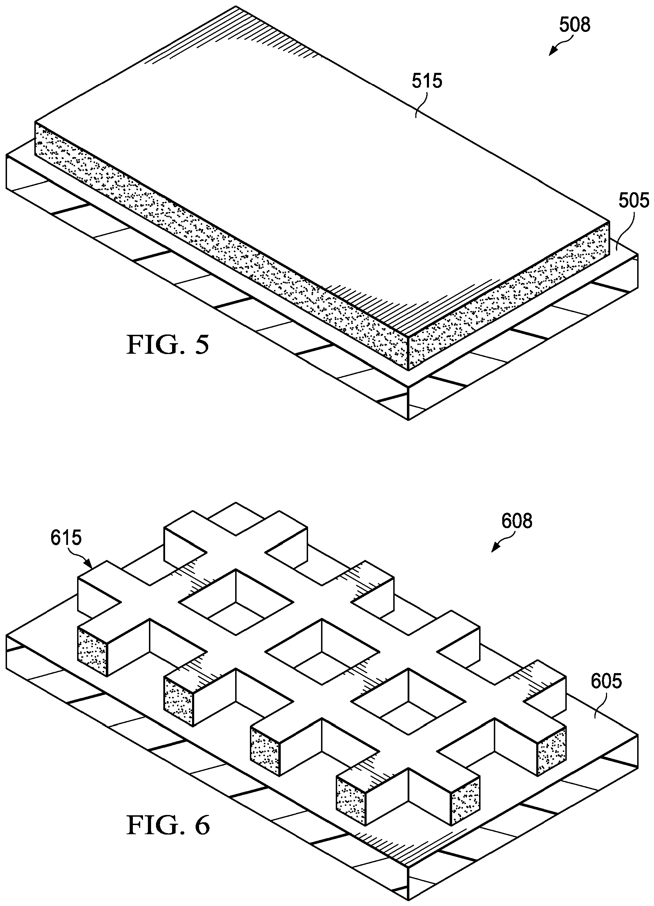

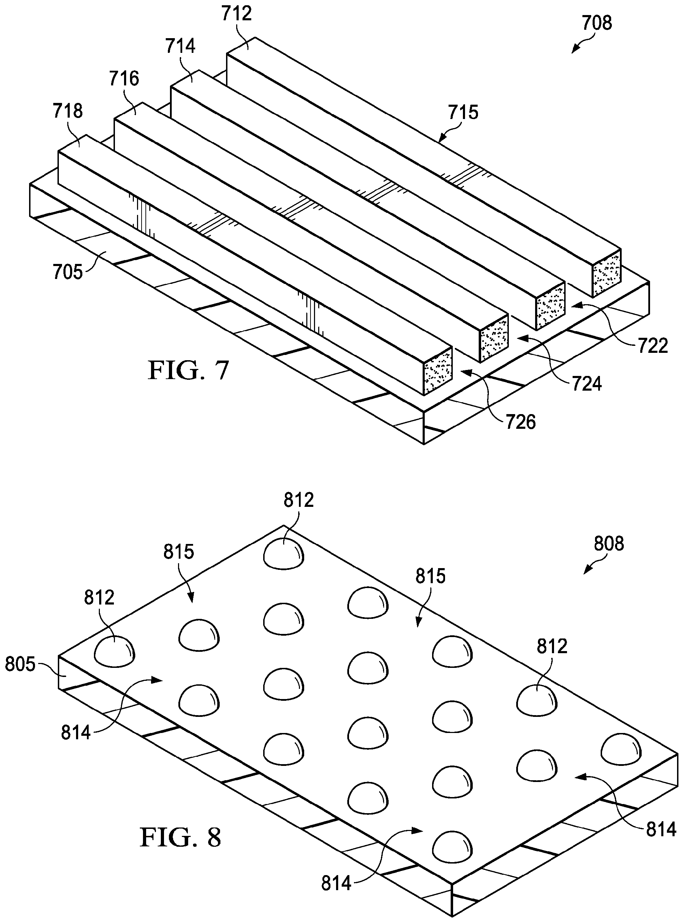

The adhesive may be applied as a continuous coating on each tissue interface layer to form the bonding layer. The bonding strength of the bonding layer may be an effective bonding strength that is proportional to the product of the adhesive bonding strength and a portion of at least one of the first interface layer or the second interface layer that is covered by the adhesive. The effective bonding strength of the adhesive may be adjusted further by printing or coating a pattern of adhesive on the tissue interface layer to increase or decrease the exposed area contacting a surface of the tissue interface layer. The proportionality of the effective bonding strength may be dependent on the shape and dimensions of the pattern as illustrated more specifically in Figures 6 and 7. The effective bonding strength also may be adjusted by utilizing localized spot bonding of the adhesive to the tissue interface layer. Instead of coating a pattern of adhesive on the tissue interface layer, the effective bonding strength may be adjusted by continuous coating a tissue interface layer that has a textured surface as described above. In one example embodiment, the effective bonding strength may be designed to be between about between about 20N per 20mm² and about 30N per 20mm² when the adhesive bonding strength is greater than about 20N per 20mm² when cured. In another example embodiment, the effective bonding strength may be designed to be between about between about 20N per 20mm² and about 30N per 20mm² when the adhesive bonding strength is greater than about 30N per 20mm² when cured.

Two or more tissue interface layers also may be bonded together lightly by welding such that the bond between a first interface layer and a second interface layer is sufficiently strong to hold them together, but weak enough to allow them to be separated or detached from each other when pulled apart by a user. In one embodiment, a local heat source may be used to create a weak-weld to heat-stake the first interface layer and the second interface layer so that they will readily detach without damaging either one of the interface layers. Such local heat sources may include, for example, lasers, ultrasonic transmitters, radio frequency (RF) transmitters, and directed heat sources using air. Bonding also may be accomplished by using mechanical interlocking techniques that are sufficiently strong to hold a first interface layer and a second interface layer together, but weak enough to allow them to be separated or detached from each other when pulled apart by a user. Such mechanical interlocking techniques may include structures having male and female components associated with opposing tissue interface layers that mechanically attached to each other when the tissue interface layers are compressed together. Such embodiments are described in more detail below. Bonding also may be accomplished by using any combination of these techniques including utilizing an adhesive.

As discussed herein, the tissue interface 108 may comprise two or more tissue interface layers bonded together lightly with one or more bonding layers. Referring to Figure 2, a tissue interface 208 is another example embodiment of the tissue interface 108. The tissue interface 208 comprises a first tissue interface layer 205, a second tissue interface layer 210, and a bonding layer 215 disposed between the first tissue interface layer 205 and the second tissue interface layer 210. The first and second interface layers 205, 210 may have all of the same or similar structures or characteristics with respect to the tissue interface layers described above in more detail. For example, the first and second interface layers 205, 210 may be bonded together lightly such that the bonding layer 215 is sufficiently strong to hold the first tissue interface layer 205 and the second interface layer 210 together without separating, but weak enough to allow the first tissue interface layer 205 to be separated or detached from the second tissue interface layer 210 when pulled apart by a user. This allows a user to add or remove the second tissue interface layer 210 to or from the first tissue interface layer 205, or any number of tissue interface layers, to adjust the height of the tissue interface 108 in order to accommodate the depth of a tissue site. The first and second tissue interface layers 205, 210 may have different thicknesses and a variety of shapes. In one example embodiment, the first and second tissue interface layers 205, 210 may have a thickness that is less than about 10 mm. In another example embodiment, the first and second tissue interface layers 205, 210 may have a thickness between about 1.0 mm and about 10 mm. The first and second tissue interface layers 205, 210 may comprise one or more materials that have a variety of shapes including, for example, materials having a sheet-like shape or thicker materials having more of a three-dimensional shape. The first and second tissue interface layers 205, 210 may have a variety of geometric shapes such as circular shapes or rectangular shapes, or a variety of irregular shapes.

In one example embodiment, the bonding layer 215 may be an adhesive that may have all of the same or similar structures or characteristics with respect to the adhesives described above in more detail. Although the bonding layer 215 may be sufficiently strong to hold the first and second interface layers 205, 210 together without separating, the bonding layer 215 may be even stronger to hold the first and second interface layers 205, 210 together in a static position relative to each other, while still being weak enough to allow the first and second interface layers 205, 210 to be separated from one another when pulled apart by a user. The bonding strength of the bonding layer 215 may be adjusted by printing or coating a pattern of adhesive on the tissue interface layer to increase or decrease the exposed area contacting the tissue interface layer as shown in Figures 5-7 described in more detail below to achieve a desired effective bonding strength. The effective bonding strength also may be adjusted by utilizing localized spot bonding of the adhesive to the tissue interface layer as shown in Figure 8 described in more detail below.

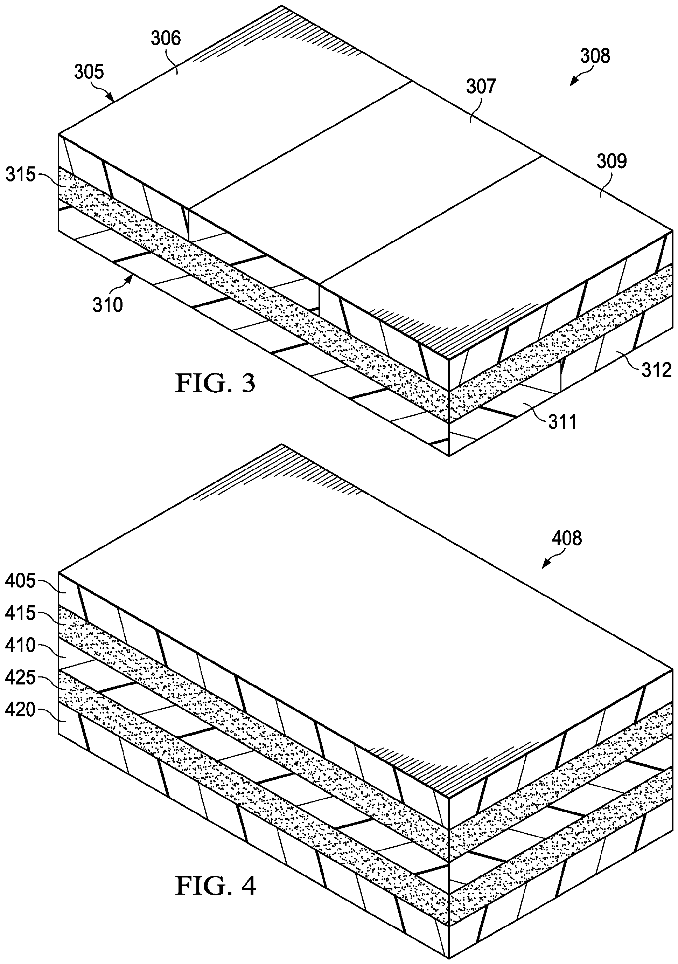

In some embodiments, each of the tissue interface layers may comprise two or more tissue interface layer segments. Each of the tissue interface layer segments may be configured to be separated in a predetermined order relative to the other tissue interface layer segments in the same tissue interface layer. Referring to Figure 3, a tissue interface 308 is another example embodiment of the tissue interface 108 and the tissue interface 208. The tissue interface 308 comprises a first tissue interface layer 305, a second tissue interface layer 310, and a bonding layer 315 disposed between the first tissue interface layer 305 and the second tissue interface layer 310. The first and second interface layers 305, 310 may have all of the same or similar structures or characteristics with respect to the tissue interface layers described above in more detail. The first tissue interface layer 305 may comprise tissue interface layer segments 306, 307, and 309 that may be separately or individually separable from the remainder of the tissue interface 308 across a length of the tissue interface 308. Similarly, the second tissue interface layer 310 may comprise tissue interface layer segments 311 and 312 that may be separately or individually separable from the remainder of the tissue interface 308 across a width of the tissue interface 308. Thus, the individual tissue interface layer segments of each tissue interface layer may be separated individually from the opposing tissue interface layer as discussed herein. For example, the tissue interface layer segment 309 may be separated from the other tissue interface layer segments 306, 307, and ultimately separated from the tissue interface layer 310, when pulled away by a user exerting enough force to overcome the strength of the bonding layer 315. It should be understood that although the individual tissue interface layer segments have rectangular shapes, one or more of the tissue interface layer segments may have different sizes and shapes to form the size and shape desired for the tissue interface layer itself.

As indicated above, two or more tissue interface layers may be bonded together lightly so that the bonding layer is sufficiently strong to hold together a first interface layer and a second interface layer, but weak enough to allow the first tissue interface layer to be separated or detached from the second tissue interface layer when pulled apart by a user. In one example embodiment, a tissue interface may have a plurality of tissue interface layers bonded together with a plurality of bonding layers. Referring to Figure 4, a tissue interface 408 is another example embodiment substantially similar to the tissue interfaces 108 and 208, but comprises an additional tissue interface layer and a bonding layer. One skilled in the art would understand that any number of tissue interface layers and bonding layers may be utilized to achieve the desired thickness as described above. The tissue interface 408 comprises a first tissue interface layer 405, a second tissue interface layer 410, and a third tissue interface layer 420. The tissue interface 408 further comprises a first bonding layer 415 disposed between the first tissue interface layer 405 and the second tissue interface layer 410, and a second bonding layer 425 disposed between the second tissue interface layer 410 and the third tissue interface layer 420. The tissue interface layers 405, 410, and 420 may have all of the same or similar structures or characteristics with respect to the tissue interface layers described above in more detail. For example, the tissue interface layers may be bonded together lightly such that the bonding layers 415, 425 are sufficiently strong to hold the tissue interface layers together without separating as described above, but weak enough to allow the tissue interface layers to be separated or detached from each other when pulled apart by a user as also described above. This allows a user to add or remove any number of tissue interface layers to or from each other to adjust the height of the tissue interface 408 in order to accommodate the depth of a tissue site. The tissue interface layers may themselves have different thicknesses and a variety of shapes. In one example embodiment, the tissue interface layers may have a thickness that is less than about 10 mm. In another example embodiment, the tissue interface layers may have a thickness between about 1.0 mm and about 10 mm.

The tissue interface layers 405, 410, and 420 may be separated or detached from each other when pulled apart by a user in any order when pulling with a force exceeding the bond strength of either one of the bonding layers 415 and 425. In some embodiments, the bond strength of the bonding layers 415 and 425 may be substantially equal. In other embodiments, the bond strength of one of the bonding layers may be weaker than the bond strength of the other bonding layer so that tissue interface layers adjacent the bonding layer having the weaker bond strength separate or detached from each other before the tissue interface layers adjacent the bonding layer having the stronger bond strength when pulled apart by a user. For example, the bond strength of the bonding layer 415 may be weaker than the bond strength of the bonding layer 425 so that tissue interface layers 405 and 410 adjacent the bonding layer 415, which has the weaker bond strength, separate or detached from each other before the tissue interface layers 410 and 420 adjacent the bonding layer 425, which has the stronger bond strength. In yet another example, the bond strength of the bonding layer 425 may be weaker than the bond strength of the bonding layer 415 so that tissue interface layers 420 and 410 adjacent the bonding layer 425, which has the weaker bond strength, separate or detached from each other before the tissue interface layers 410 and 405 adjacent the bonding layer 415, which has the stronger bond strength. It should be understood that a tissue interface may be constructed to have any number of tissue interface layers held together by interleaving bonding layers having a gradient of bond strengths so that the tissue interface layers may be separated in a predetermined order depending on the bond strength gradient.

As indicated above, a bonding layer may have an effective bonding strength that may be adjusted by printing or coating a pattern of adhesive on the tissue interface layer to increase or decrease the exposed area contacting the tissue interface layer. In one example embodiment, the bonding layer may be a continuous coating covering the entire surface of the adjacent tissue interface layers 205, 210 as shown in Figure 2, wherein the bond strength may be the adhesive bonding strength as described above. In other example embodiments, a bonding layer may be a pattern covering only a portion of the surface of a tissue interface layer that effectively reduces the bond strength, i.e., the effective bonding strength described above, but is still strong enough to couple one tissue interface layer to another tissue interface layer. Such patterns may have a portion of the entire surface area of the pattern that comes in contact with the corresponding portion of the surface of the tissue interface layer. The more surface area that a bonding layer covers on a surface of a tissue interface layer, the greater the effective bonding strength of the bonding layer to prevent two tissue interface layers from being detached or separated from each other. The effective bonding strength may be the adhesive bonding strength when the bonding layer contacts the entire surface area of a tissue interface layer.

As indicated above, a tissue interface may have a plurality of tissue interface layers bonded together with a plurality of bonding layers and is not limited to a tissue interface having a specific number of tissue layers such as, for example, the tissue interface 408 having three tissue interface layer separated by two bonding layers. A tissue interface may comprise a plurality of tissue interface layers and a plurality of bonding layers interleaved between each of the plurality of tissue interface, wherein the bonding layers may have a bonding strength for coupling the tissue interface layers together. The bonding strength is sufficiently weak for allowing each of the plurality of tissue interface layers to be separated from each other when a force greater than the bonding strength is applied to pull each of the tissue interface layers away from the other tissue interface layers in order to reduce the overall thickness of the tissue interface to better conform to the depth of the tissue site. Moreover, the bonding layers may retain their tackiness for each of the tissue interface layers to be added back to the tissue interface as necessary. The bonding strength of each of the plurality of bonding layers may be different from the bonding strength of the other bonding layers so that the tissue interface layers may be separated in a predetermined order as they are separated from the tissue interface.

Referring to Figure 5, a tissue interface 508 is another example embodiment of the tissue interface 108. The tissue interface 508 comprises a first tissue interface layer 505, a second tissue interface layer (not shown), and a bonding layer 515 disposed on the first tissue interface layer 505. The first interface layer 505 (and the second interface layer) may have all of the same or similar structures or characteristics with respect to the tissue interface layers described above in more detail. The bonding layer 515 covers substantially the entire surface of the first tissue interface layer 505 except for a perimeter portion of the first tissue interface layer 505. It should be understood that the second tissue interface layer may be coupled to the first tissue interface layer 505 using the bonding layer 510 and may be separated or detached from the first tissue interface layer 505 when the user pulls a second tissue interface layer with a force that exceeds the maximum bond strength of the bonding layer 515. The perimeter portion of the first tissue interface layer 505 may be provided for a user to easily grasp the first interface layer 505 to pull and separate the two tissue interface layers. Because the bonding layer 515 does not cover the entire surface of the first tissue interface layer 505, the amount of force necessary for the user to separate the two interface layers would be a little less than the maximum bond strength of the bonding layer 515.

Figure 6 illustrates another example embodiment of a tissue interface that may be the same as or similar to the tissue interface 108 but, more specifically, a tissue interface comprising a first tissue interface layer and a bonding layer that does not cover the entire surface of a tissue interface layer. A tissue interface 608 comprises a first tissue interface layer 605, a second tissue interface layer (not shown), and a bonding layer 615 disposed on the first tissue interface layer 605. The first interface layer 605 (and the second interface layer) may have all of the same or similar structures or characteristics with respect to the tissue interface layers described above in more detail. The bonding layer 615 is formed in the shape of a grid pattern that covers a surface area of the first tissue interface layer 605 that is less than the entire surface area of the surface of the first tissue interface layer 605. In one example embodiment, the component of the pattern may cover about 50% of the surface area of the first tissue interface layer 605 such that the effective bonding strength of the bonding layer 615 is about half of the maximum bond strength. It should be understood that components of the pattern may be sized and oriented to achieve the effective bonding strength that is desired. As indicated above, the second tissue interface layer may be coupled to the first tissue interface layer 605 by positioning the second tissue interface layer on top of the bonding layer 615 in a similar fashion, and later may be separated or detached from the first tissue interface layer 605 when a user pulls the second tissue interface layer with a force that exceeds the effective bonding strength of the bonding layer 615. In some embodiments, when the bonding layer 615 is positioned on a surface of the first tissue interface layer 605, a portion of the surface of the first tissue interface layer 605 may not be covered with the bonding layer 615 so that a user may easily grasp the portion of the surface of the first tissue interface layer 605.

Figure 7 illustrates another example embodiment of a tissue interface that may be the same as or similar to the tissue interface 108 but, more specifically, a tissue interface comprising a first tissue interface layer and a bonding layer that does not cover the entire surface of a tissue interface layer. A tissue interface 708 comprises a first tissue interface layer 705, a second tissue interface layer (not shown), and a bonding layer 715 disposed on the first tissue interface layer 705. The first interface layer 705 (and the second interface layer) may have all of the same or similar structures or characteristics with respect to the tissue interface layers described above in more detail. In this example embodiment, the bonding layer 715 is formed by a plurality of linear segments 712, 714, 716, and 718 aligned in parallel at select locations on a surface of the first tissue interface layer 705 and forming open spaces 722, 724, and 726 between them on the surface of the first tissue interface layer 705. The linear segments 712, 714, 716, and 718 cover a surface area of the first tissue interface layer 705 that is less than the entire surface area of the surface of the first tissue interface layer 705. In one example embodiment, the linear segments may cover about 50% of the surface area of the first tissue interface layer 705 such that the effective bonding strength of the bonding layer 715 is about half of the maximum bond strength. The bonding layer segments 712, 714, 716, and 718 may be sized and aligned to achieve the effective bonding strength that is desired. As indicated above, the second tissue interface layer may be coupled to the first tissue interface layer 705 by positioning the second tissue interface layer on top of the bonding layer 715 in a similar fashion, and later may be separated or detached from the first tissue interface layer 705 when a user pulls the second tissue interface layer with a force that exceeds the effective bonding strength of the bonding layer 715. As indicated above, the linear segments 712, 714, 716, and 718 may be sized and aligned to achieve the effective bonding strength that is desired. In this example embodiment, the linear segments 712, 714, 716, and 718 are aligned in parallel so that the effective bonding strength may be about half the maximum bond strength when the user pulls the second tissue interface layer away from the first tissue interface layer 705 in a direction parallel to the linear segments. However, the effective bonding strength may be greater than half of the maximum bond strength when the user pulls the second interface layer away from the first tissue interface layer 705 in a direction perpendicular to the linear segments.

Figure 8 illustrates another example embodiment of a tissue interface that may be the same as or similar to the tissue interface 108 but, more specifically, a tissue interface comprising a first tissue interface layer and a bonding layer that does not cover the entire surface of a tissue interface layer. A tissue interface 808 comprises a first tissue interface layer 705, a second tissue interface layer (not shown), and a bonding layer 815 disposed on the first tissue interface layer 805. The first interface layer 805 (and the second interface layer) may have all of the same or similar structures or characteristics with respect to the tissue interface layers described above in more detail. In this example embodiment, the bonding layer 815 is formed by a plurality of spot segments 812 formed in a pattern at select locations on a surface of the first tissue interface layer 805, and forming open spaces 814 between them on the surface of the first tissue interface layer 805. The spot segments 812 cover a surface area of the first tissue interface layer 805 that is less than the entire surface area of the surface of the first tissue interface layer 805. In one example embodiment, the component of the pattern may cover about 50% of the surface area of the first tissue interface layer 805 such that the effective bonding strength of the bonding layer 815 is about half of the maximum bond strength. The spot segments 812 may be sized and aligned to achieve the effective bonding strength that is desired. As indicated above, the second tissue interface layer may be coupled to the first tissue interface layer 805 by positioning the second tissue interface layer on top of the bonding layer 815 in a similar fashion, and later may be separated or detached from the first tissue interface layer 805 when a user pulls the second tissue interface layer with a force that exceeds the effective bonding strength of the bonding layer 815.

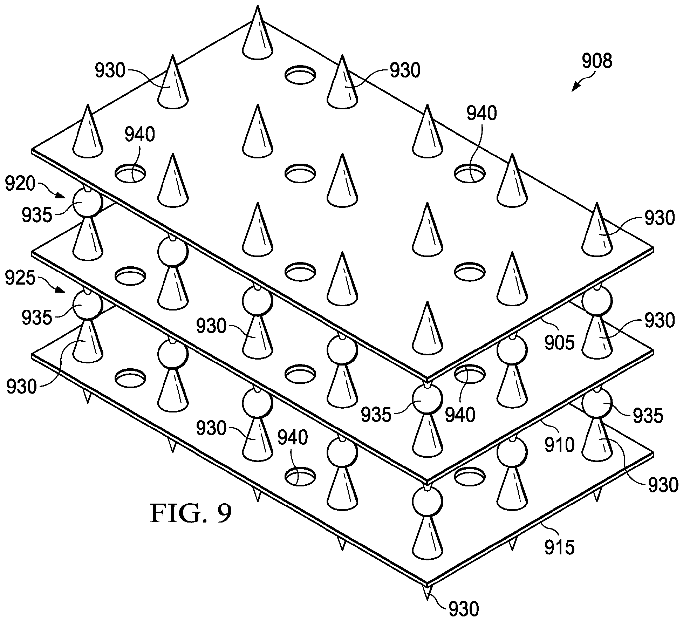

In some embodiments of a tissue interface, the tissue interfaces may have a bed-of-nails structure. Referring to Figure 9, an example embodiment of a tissue interface 908 is shown that may be the same as or similar to the tissue interface 108 and the tissue interface 408. The tissue interface 908 may have a bed-of-nails structure including a first tissue interface layer 905, a second tissue interface layer 910, and a third tissue interface layer 915. Each of the tissue interface layers may include a plurality of protrusions 930 extending from surfaces of the tissue interface layers such as, for example, spikes or nails. One surface of either one of the tissue interface layers 905, 915 facing a tissue site may include protrusions 930 to provide abrasion on a surface of the tissue site. In some embodiments, each of the tissue interface layers may include one or more perforations 940 extending at least partially through a tissue interface layer. The tissue interface 908 may also include a first bonding layer 920 and a second bonding layer 925. The first bonding layer 920 and the second bonding layer 925 may include a plurality of bonding layer spot segments 935 positioned at select locations on a surface of a tissue interface layer such as, for example, on each protrusion 930 or at the end of each protrusion 930 extending from the surface of a tissue interface layer. For example, each bonding layer spot segment 935 of the first bonding layer 920 may bond or couple the first tissue interface layer 905 to the second tissue interface layer 910 at the protrusions 930, and each bonding layer spot segment 935 of the second bonding layer 925 may bond or couple the second tissue interface layer 910 to the third tissue interface layer 915 at the protrusions 930. Because the bonding layer spot segments 935 are positioned on the protrusions 930, spaces may be formed between tissue interface layers. Thus, in some embodiments, the perforations 940 and the spaces formed between the tissue interface layers may facilitate fluid communication through the tissue interface 908 when, for example, negative pressure is applied to the tissue interface 908.

When a force acting on the first tissue interface layer 905 reaches or exceeds an effective bonding strength of the first bonding layer 920, the first bonding layer 920 may release the first tissue interface layer 905 from the from the second tissue interface layer 910 and the third tissue interface layer 915. Similarly, when a force acting on the third tissue interface layer 915 reaches or exceeds an effective bonding strength of the second bonding layer 925, the second bonding layer 925 may release the third tissue interface layer 915 from the first tissue interface layer 905 and the second tissue interface layer 910. When a force acting on the second tissue interface layer 910 reaches or exceeds an effective bonding strength of the first bonding layer 920 and the second bonding layer 925, the second bonding layer 925 may release the second tissue interface layer 910 from the first tissue interface layer 905 or the second tissue interface layer 910. For example, the first bonding layer 920 and the second bonding layer 925 may release the second tissue interface layer 910 from the second tissue interface layer 910 to be displaced along a surface of the first tissue interface layer 905 and a surface of the third tissue interface layer 915. In various embodiments, the second bonding layer 925 may release the second tissue interface layer 910 from the second tissue interface layer 910 and the third tissue interface layer 915 to be displaced away the first tissue interface layer 905. Similarly, the first bonding layer 920 may release the second tissue interface layer 910 from the second tissue interface layer 910 and the first tissue interface layer 905 to be displaced away from the third tissue interface layer 915.

In some embodiments, an effective bonding strength of the first bonding layer 920 may be greater than or less than an effective bonding strength of the second bonding layer 920. For example, when an effective bonding strength of the first bonding layer 920 is greater than an effective bonding strength of the second bonding layer 925, a force that is less than the effective bonding strength of the first bonding layer 920 and greater than the effective bonding strength of the second bonding layer 925, is acting in a direction along the surface of the second tissue interface layer 910, and is acting on the second tissue interface layer 910 may cause the first tissue interface layer 905 and the second tissue interface layer 910 to be displaced together. As another example, when an effective bonding strength of the first bonding layer 920 is less than an effective bonding strength of the second bonding layer 925, a force that is greater than the effective bonding strength of the first bonding layer 920 and less than the effective bonding strength of the second bonding layer 925, is acting in a direction along the surface of the second tissue interface layer 910, and is acting on the second tissue interface layer 910 may cause the second tissue interface layer 910 and the third tissue interface layer 915 to be displaced together. It should be understood that when a force acting on the second tissue interface layer 910 is less than both the effective bonding strength of the first bonding layer 920 and the effective bonding strength of the second bonding layer 925, the second tissue interface layer 910 may not displace relative to the first tissue interface layer 905 or the third tissue interface layer 915. It should also be understood that when a force acting on the second tissue interface layer 910 is greater than both the effective bonding strength of the first bonding layer 920 and an effective bonding strength of the second bonding layer 925, the second tissue interface layer 910 may displace relative to both the first tissue interface layer 905 and the third tissue interface layer 915.

As discussed herein, bonding layers may include mechanical interlocking systems. Mechanical interlocking systems may include structures that can bond or couple a first tissue interface layer together with a second tissue interface layer when the first tissue interface layer and the second tissue interface layer are compressed against each other. Mechanical interlocking systems may also include structures that can detach a first tissue interface layer from a second tissue interface layer after the first tissue interface layer and the second tissue interface layer are coupled and bonded together. Thus, in some embodiments, mechanical interlocking system may permit a tissue interface layer to be bonded or coupled to another tissue interface layer and subsequently detached from each other for removal or adjustment of at least one tissue interface layer. When a tissue interface layer is detached from another tissue interface layer for adjustments, a mechanical interlocking system may allow the tissue interface layer to be rebonded or recoupled to the other tissue interface layer.

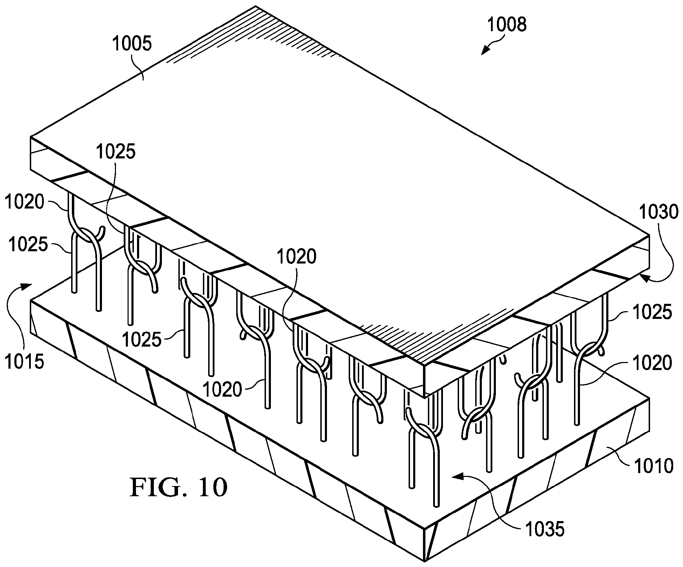

In some embodiments, a bonding layer may comprise a mechanical interlocking system that includes a hook and loop structure. Figure 10 illustrates a tissue interface 1008 that may be the same as or similar to the tissue interface 108 illustrated in Figure 1. As shown in Figure 10, the tissue interface 1008 may include a first tissue interface layer 1005, a second tissue interface layer 1010, and a bonding layer 1015. The bonding layer 1015 may include a hook and loop structure comprising a plurality of hooks and a plurality of loops. For example, a plurality of hooks 1020 and a plurality of loops 1025 may extend from a surface 1030 of the first tissue interface layer 1005 and a plurality of hooks 1020 and a plurality of loops 1025 may extend from a surface 1035 of the second tissue interface layer 1010. When the first tissue interface layer 1005 is compressed against the second tissue interface layer 1010, the hooks 1020 extending from the surface 1030 may engage with the loops 1025 extending from the surface 1035 and hook through the loops 1025. Similarly, when the first tissue interface layer 1005 is compressed against the second tissue interface layer 1010, the hooks 1020 extending from the surface 1035 may engage with the loops 1025 extending from the surface 1030 and hook through the loops 1025. The hooks 1020 hooked through the loops 1025 may bond or couple the first tissue interface 1005 with the second tissue interface 1010. The hooks 1020 and the loops 1025 may include a flexible or elastic material to allow the hooks 1020 and loops 1025 to bend or flex to release the hooks 1020 from the loops 1025 when an effective bonding strength of the bonding layer 1015 is met or exceeded. The hooks 1020 and the loops 1025 may also return to their original shapes after bending or flexing and after the first tissue interface layer 1005 and the second tissue interface layer 1010 detach so that the first tissue interface layer 1005 and the second tissue interface layer 1010 may rebond or recouple to each other. In some embodiments, when a tissue interface has a bonding layer that includes a mechanical interlocking system and when a first tissue interface layer of the tissue interface is detached from a second tissue interface layer of the tissue interface, a portion of the mechanical interlocking system extending from a surface of the first tissue interface layer or a portion of the mechanical interlocking system extending from a surface of the second tissue interface layer may be used as a granulation member to generate granulation on a surface of a tissue site when the portion of the mechanical interlocking system is in direct contact with the surface of the tissue site.

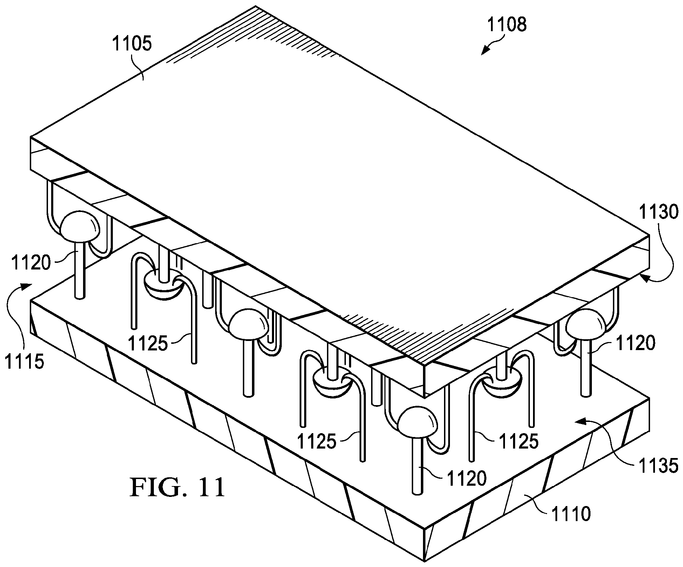

In some embodiments, a bonding layer may comprise a mechanical interlocking system that includes a dome and spike structure. Figure 11 illustrates a tissue interface 1108 that may be the same as or similar to the tissue interface 108 illustrated in Figure 1. As shown in Figure 11, the tissue interface 1108 may include a first tissue interface layer 1105, a second tissue interface layer 1110, and a bonding layer 1115. The bonding layer 1115 may include a dome and spike structure comprising a plurality of domes and a plurality of spikes. For example, a plurality of domes 1120 and a plurality of spikes 1125 may extend from a surface 1130 of the first tissue interface layer 1105 and a plurality of domes 1120 and a plurality of spikes 1125 may extend from a surface 1135 of the second tissue interface layer 1110. When the first tissue interface layer 1105 is compressed against the second tissue interface layer 1110, the domes 1120 extending from the surface 1130 may engage with the spikes 1125 extending from the surface 1135 so that the spikes 1125 latch on to the domes 1120. Similarly, when the first tissue interface layer 1105 is compressed against the second tissue interface layer 1110, the domes 1120 extending from the surface 1135 may engage with the spikes 1125 extending from the surface 1130 so that the spikes 1125 latch on to the domes 1120. The spikes 1125 latched on to the domes 1120 may bond or couple the first tissue interface 1105 together with the second tissue interface 1110. The domes 1120 and the spikes 1125 may include a flexible or elastic material to allow the domes 1120 and spikes 1125 to bend or flex to release the spikes 1120 from the domes 1125 when an effective bonding strength of the bonding layer 1115 is met or exceeded. The domes 1120 and the spikes 1125 may also return to their original shapes after bending or flexing and after the first tissue interface layer 1105 and the second tissue interface layer 1110 detach so that the first tissue interface layer 1105 and the second tissue interface layer 1010 may rebond or recouple to each other. As shown in Figure 11, a surface of a tissue interface layer may have a ratio of one dome for every two spikes. In various embodiments, a surface of a tissue interface layer may have two domes for every eight spikes or one dome for every nine spikes. One of ordinary skill in the art would be able to identify the various ratios of domes to spikes on a surface of a tissue interface layer to detach the tissue interface layer from another tissue interface layer as discussed herein.

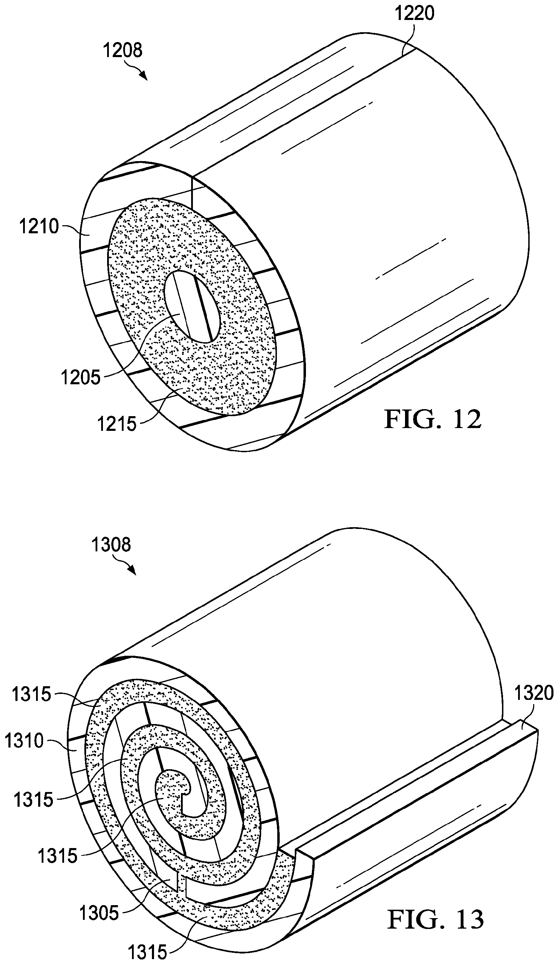

In some embodiments, a tissue interface may include a cylindrical structure. A tissue interface that includes a cylindrical structure may accommodate, for example, a tissue site that includes a tunnel wound. Figure 12 illustrates an example embodiment of a tissue interface 1208 that may be the same as or similar to the tissue interface 108 illustrated in Figure 1. As shown in Figure 12, the tissue interface 1208 may include a core interface layer 1205 and at least one circumferential interface layer 1210 positioned in a circumferential orientation around the core interface layer 1205. The circumferential interface layer 1210 may be bonded or coupled to the core interface layer 1205 with a bonding layer 1215 positioned in a circumferential orientation around the core interface layer 1205 and between the core interface layer 1205 and the circumferential interface layer 1210. When a force acting on the circumferential interface layer 1210 reaches or exceeds an effective bonding strength of the bonding layer 1215, the bonding layer 1215 may release the circumferential interface layer 1210 from the static position so that the circumferential interface layer 1210 may displace relative the core interface layer 1205. The circumferential interface layer 1210 may displace relative to the core interface layer 1205 by sliding in axial direction along the tissue interface 1208. Additionally or alternatively, the circumferential interface layer 1210 may displace relative to the core interface layer 1205 by detaching from the core interface layer 1205 in a radial direction. For example, the circumferential interface layer 1210 may be displaced relative to the core interface layer 1205 by peeling away from the core interface layer 1205 at one or more seams 1220 on the circumferential interface layer 1210. Similarly, when a force acting on the core interface layer 1205 reaches or exceeds an effective bonding strength of the bonding layer 1215, the bonding layer 1215 may release the core interface layer 1205 from the static position so that the core interface layer 1205 may displace relative the circumferential interface layer 1210. The core interface layer 1205 may displace relative to the circumferential interface layer 1210 by sliding in axial direction along the tissue interface 1208.

In some embodiments, a tissue interface may include a coiled structure. At least similar to tissue interfaces that have cylindrical structures, a tissue interface that includes a coiled structure may accommodate, for example, a tissue site that includes a tunnel wound. Figure 13 illustrates an example embodiment of a tissue interface 1308 that may be the same as or similar to the tissue interface 108 illustrated in Figure 1. As shown in Figure 13, the tissue interface 1308 may include an inner interface layer 1305 coiled around a center axis of the tissue interface 1308 and at least one outer interface layer 1310 coiled around the inner interface layer 1305. A bonding layer 1315 may be positioned between the inner interface layer 1305 and the outer interface layer 1310 to bond or couple the outer interface layer 1310 to the inner interface layer 1305. Additionally or alternatively, the bonding layer 1315 may positioned between folds of the inner interface layer 1305 to retain or hold the inner interface layer 1305 in a coiled orientation. Further, the bonding layer 1315 may be positioned between folds of the outer interface layer 1310 to hold or retain the coiled structure of the tissue interface 1308. When a force acting on the outer interface layer 1310 reaches or exceeds an effective bonding strength of the bonding layer 1315, the bonding layer 1315 may release the outer interface layer 1310 from the static position so that the outer interface layer 1310 may displace relative the inner interface layer 1305. The outer interface layer 1310 may displace relative to the inner interface layer 1305 by sliding in axial direction along the tissue interface 1308. Additionally or alternatively, the outer interface layer 13 10 may displace relative to the inner interface layer 1305 by detaching from the inner interface layer 1305 in a radial direction. For example, the outer interface layer 1310 may be displaced relative to the inner interface layer 1305 by peeling away from the inner interface layer 1305 at an end 1320 of the outer interface layer 1310.

The systems, apparatuses, and methods described herein may provide significant advantages. For example, the tissue interface (such as wound filler) may reduce trauma and facilitate ease of removal when treating both deep and shallow wounds. The tissue interface encourages granulation without the disadvantage of tissue ingrowth resulting in pain or discomfort upon removal.

While shown in a few illustrative embodiments, a person having ordinary skill in the art will recognize that the systems, apparatuses, and methods described herein are susceptible to various changes and modifications. Moreover, descriptions of various alternatives using terms such as or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 102, the container 112, or both may be eliminated or separated from other components for manufacture or sale. In other example configurations, the controller 110 may also be manufactured, configured, assembled, or sold independently of other components.

## Claims

1. A tissue interface configured to be positioned at a tissue site, the tissue interface comprising:
a first tissue interface layer (205, 305, 405, 505, 605, 705, 805, 905, 1005, 1105) having a surface adapted to promote granulation of tissue when placed on tissue under negative pressure; a second tissue interface layer (210, 310, 410, 910, 1010, 1110); and
a first bonding layer (215, 315, 415, 515, 615, 715, 815, 915) positioned between the first tissue interface layer (205, 305, 405, 505, 605, 705, 805, 905, 1005, 1105) and the second tissue interface layer (210, 310, 410, 910, 1010, 1110) and having a bonding strength for coupling the first tissue interface layer (205, 305, 405, 505, 605, 705, 805, 905, 1005, 1105) to the second tissue interface layer (210, 310, 410, 910, 1010, 1110), wherein the bonding strength is sufficiently weak for allowing the first tissue interface layer (205, 305, 405, 505, 605, 705, 805, 905, 1005, 1105) to be separated from the second tissue interface layer (210, 310, 410, 910, 1010, 1110) when a force greater than the bonding strength is applied to pull the first tissue interface layer (205, 305, 405, 505, 605, 705, 805, 905, 1005, 1105) away from the second tissue interface layer (210, 310, 410, 910, 1010, 1110), wherein the first bonding layer (215, 315, 415, 515, 615, 715, 815, 915) comprises an adhesive,
**characterized in that** the bonding strength of the first bonding layer (215, 315, 415, 515, 615, 715, 815, 915) is between 1.0N per mm² and 1.5N per mm² when cured.

2. The tissue interface of Claim 1, wherein the second tissue interface layer (210, 310, 410, 910, 1010, 1110) has a surface adapted to promote granulation of tissue when placed on tissue under negative pressure.

3. The tissue interface of Claim 1, wherein at least one of the first tissue interface layer (205, 305, 405, 505, 605, 705, 805, 905, 1005, 1105) and the second tissue interface layer (210, 310, 410, 910, 1010, 1110) comprises at least one of a composite sheet, a woven gauze, a polymer extruded gauze, a welded polymer mesh, and a polymer bubble wrap material.

4. The tissue interface of Claim 1, wherein at least one of the first tissue interface layer (205, 305, 405, 505, 605, 705, 805, 905, 1005, 1105) and the second tissue interface layer (210, 310, 410, 910, 1010, 1110) comprises a sheet having a thickness of less than 10.0 mm.

5. The tissue interface of claim 1, further comprising:
a third tissue interface layer (420, 915); and
a second bonding layer (425, 925) positioned between the third tissue interface layer (420, 915) and the second tissue interface layer (210, 310, 410, 910, 1010, 1110) and having a bonding strength for coupling the third tissue interface layer (420, 915) to the second tissue interface layer (210, 310, 410, 910, 1010, 1110), wherein the bonding strength is sufficiently weak for allowing the third tissue interface layer (420, 915) to be separated from the second tissue interface layer (210, 310, 410, 910, 1010, 1110) when a force greater than the bonding strength is applied to pull the third tissue interface layer (420, 915) away from the second tissue interface layer (210, 310, 410, 910, 1010, 1110).

6. The tissue interface of Claim 5, wherein the second bonding layer (425, 925) comprises an adhesive.

7. The tissue interface of Claim 6, wherein the bonding strength of the second bonding layer (425, 925) is between 20N per 20mm² and 30N per 20mm² when cured.

8. The tissue interface of Claim 5, wherein the bonding strength of the first bonding layer (415, 915) and the second bonding layer (425, 925) are different values.

9. The tissue interface of Claim 5, wherein each one of the first tissue interface layer (405, 905), the second tissue interface layer (410, 910), and the third tissue interface layer (420, 915) comprises a sheet having a thickness of less than 10.0 mm.

## Patentansprüche

1. Gewebeschnittstelle, die dazu konfiguriert ist, an einer Gewebestelle positioniert zu werden, wobei die Gewebeschnittstelle umfasst:
- eine erste Gewebe-Schnittstellenschicht (205, 305, 405, 505, 605, 705, 805, 905, 1005, 1105) mit einer Oberfläche, die dazu ausgelegt ist, um die Granulation von Gewebe zu fördern, wenn sie unter Unterdruck auf Gewebe platziert wird;
- eine zweite Gewebe-Schnittstellenschicht (210, 310, 410, 910, 1010, 1110); und
- eine erste Haftschicht (215, 315, 415, 515, 615, 715, 815, 915), die zwischen der ersten Gewebe-Schnittstellenschicht (205, 305, 405, 505, 605, 705, 805, 905, 1005, 1105) und der zweiten Gewebe-Schnittstellenschicht (210, 310, 410, 910, 1010, 1110) positioniert ist und eine Haftfestigkeit zum Koppeln der ersten Gewebe-Schnittstellenschicht (205, 305, 405, 505, 605, 705, 805, 905, 1005, 1105) mit der zweiten Gewebe-Schnittstellenschicht (210, 310, 410, 910, 1010, 1110) aufweist, wobei die Haftfestigkeit ausreichend schwach ist, um der ersten Gewebe-Schnittstellenschicht (205, 305, 405, 505, 605, 705, 805, 905, 1005, 1105) zu ermöglichen, von der zweiten Gewebe-Schnittstellenschicht (210, 310, 410, 910, 1010, 1110) getrennt zu werden, wenn eine Kraft ausgeübt wird, die größer als die Haftfestigkeit ist, um die erste Gewebe-Schnittstellenschicht (205, 305, 405, 505, 605, 705, 805, 905, 1005, 1105) weg von der zweiten Gewebe-Schnittstellenschicht (210, 310, 410, 910, 1010, 1110) zu ziehen, wobei die erste Haftschicht (215, 315, 415, 515, 615, 715, 815, 915) einen Klebstoff umfasst,
**dadurch gekennzeichnet, dass** die Haftfestigkeit der ersten Haftschicht (215, 315, 415, 515, 615, 715, 815, 915) im ausgehärteten Zustand zwischen 1,0 N pro mm² und 1,5 N pro mm² beträgt.

2. Gewebeschnittstelle nach Anspruch 1, wobei die zweite Gewebe-Schnittstellenschicht (210, 310, 410, 910, 1010, 1110) eine Oberfläche aufweist, die dazu ausgelegt ist, um die Granulation von Gewebe zu fördern, wenn sie unter Unterdruck auf Gewebe platziert wird.

3. Gewebeschnittstelle nach Anspruch 1, wobei mindestens eine von der ersten Gewebe-Schnittstellenschicht (205, 305, 405, 505, 605, 705, 805, 905, 1005, 1105) und der zweiten Gewebe-Schnittstellenschicht (210, 310, 410, 910, 1010, 1110) mindestens eines von einer Verbundfolie, einer gewebten Gaze, einer polymerextrudierten Gaze, einem geschweißten Polymernetz und einem Polymer-Luftpolsterfolienmaterial umfasst.

4. Gewebeschnittstelle nach Anspruch 1, wobei mindestens eines von der ersten Gewebe-Schnittstellenschicht (205, 305, 405, 505, 605, 705, 805, 905, 1005, 1105) und der zweiten Gewebe-Schnittstellenschicht (210, 310, 410, 910, 1010, 1110) eine Lage mit einer Dicke von weniger als 10,0 mm umfasst.

5. Gewebeschnittstelle nach Anspruch 1, weiter umfassend:
- eine dritte Gewebe-Schnittstellenschicht (420, 915); und
- eine zweite Haftschicht (425, 925), die zwischen der dritten Gewebe-Schnittstellenschicht (420, 915) und der zweiten Gewebe-Schnittstellenschicht (210, 310, 410, 910, 1010, 1110) positioniert ist und eine Haftfestigkeit zum Koppeln der dritten Gewebe-Schnittstellenschicht (420, 915) mit der zweiten Gewebe-Schnittstellenschicht (210, 310, 410, 910, 1010, 1110) aufweist, wobei die Haftfestigkeit ausreichend schwach ist, um zu ermöglichen, dass die dritte Gewebe-Schnittstellenschicht (420, 915) von der zweiten Gewebe-Schnittstellenschicht (210, 310, 410, 910, 1010, 1110) getrennt werden kann, wenn eine Kraft größer als die Haftfestigkeit ausgeübt wird, um die dritte Gewebe-Schnittstellenschicht (420, 915) weg von der zweiten Gewebe-Schnittstellenschicht (210, 310, 410, 910, 1010, 1110) zu ziehen.

6. Gewebeschnittstelle nach Anspruch 5, wobei die zweite Haftschicht (425, 925) einen Klebstoff umfasst.

7. Gewebeschnittstelle nach Anspruch 6, wobei die Haftfestigkeit der zweiten Haftschicht (425, 925) im ausgehärteten Zustand zwischen 20 N pro 20 mm² und 30 N pro 20 mm² beträgt.

8. Gewebeschnittstelle nach Anspruch 5, wobei die Haftfestigkeit der ersten Haftschicht (415, 915) und der zweiten Haftschicht (425, 925) unterschiedliche Werte sind.

9. Gewebeschnittstelle nach Anspruch 5, wobei jedes von mindestens einer von der ersten Gewebe-Schnittstellenschicht (405, 905), der zweiten Gewebe-Schnittstellenschicht (410, 910) und der dritten Gewebe-Schnittstellenschicht (420, 915) eine Lage mit einer Dicke von weniger als 10,0 mm umfasst.

## Revendications

1. Interface tissulaire configurée pour être positionnée au niveau d'un site tissulaire, l'interface tissulaire comprenant :
une première couche d'interface tissulaire (205, 305, 405, 505, 605, 705, 805, 905, 1005, 1105) présentant une surface adaptée pour favoriser une granulation de tissu lorsqu'elle est placée sur un tissu sous pression négative ;
une deuxième couche d'interface tissulaire (210, 310, 410, 910, 1010, 1110) ; et
une première couche de liaison (215, 315, 415, 515, 615, 715, 815, 915) positionnée entre la première couche d'interface tissulaire (205, 305, 405, 505, 605, 705, 805, 905, 1005, 1105) et la deuxième couche d'interface tissulaire (210, 310, 410, 910, 1010, 1110) et présentant une puissance de liaison pour coupler la première couche d'interface tissulaire (205, 305, 405, 505, 605, 705, 805, 905, 1005, 1105) à la deuxième couche d'interface tissulaire (210, 310, 410, 910, 1010, 1110), dans laquelle la puissance de liaison est suffisamment faible pour permettre à la première couche d'interface tissulaire (205, 305, 405, 505, 605, 705, 805, 905, 1005, 1105) de se séparer de la deuxième couche d'interface tissulaire (210, 310, 410, 910, 1010, 1110) lorsqu'une force supérieure à la puissance de liaison est appliquée pour tirer la première couche d'interface tissulaire (205, 305, 405, 505, 605, 705, 805, 905, 1005, 1105) à l'écart de la deuxième couche d'interface tissulaire (210, 310, 410, 910, 1010, 1110), dans laquelle la première couche de liaison (215, 315, 415, 515, 615, 715, 815, 915) comprend un adhésif,
**caractérisée en ce que** la puissance de liaison de la première couche de liaison (215, 315, 415, 515, 615, 715, 815, 915) est comprise entre 1,0 N par mm² et 1,5 N par mm² lorsqu'elle est durcie.

2. Interface tissulaire selon la revendication 1, dans laquelle la deuxième couche d'interface tissulaire (210, 310, 410, 910, 1010, 1110) présente une surface adaptée pour favoriser une granulation de tissu lorsqu'elle est placée sur un tissu sous pression négative.

3. Interface tissulaire selon la revendication 1, dans laquelle au moins l'une de la première couche d'interface tissulaire (205, 305, 405, 505, 605, 705, 805, 905, 1005, 1105) et de la deuxième couche d'interface tissulaire (210, 310, 410, 910, 1010, 1110) comprend au moins l'un d'une feuille composite, d'une gaze tissée, d'une gaze extrudée polymère, d'une maille polymère soudée et d'un matériau polymère d'emballage à bulles.

4. Interface tissulaire selon la revendication 1, dans laquelle au moins l'une de la première couche d'interface tissulaire (205, 305, 405, 505, 605, 705, 805, 905, 1005, 1105) et de la deuxième couche d'interface tissulaire (210, 310, 410, 910, 1010, 1110) comprend une feuille présentant une épaisseur inférieure à 10,0 mm.

5. Interface tissulaire selon la revendication 1, comprenant en outre :
une troisième couche d'interface tissulaire (420, 915) ; et
une seconde couche de liaison (425, 925) positionnée entre la troisième couche d'interface tissulaire (420, 915) et la deuxième couche d'interface tissulaire (210, 310, 410, 910, 1010, 1110) et présentant une puissance de liaison pour coupler la troisième couche d'interface tissulaire (420, 915) à la deuxième couche d'interface tissulaire (210, 310, 410, 910, 1010, 1110), dans laquelle la puissance de liaison est suffisamment faible pour permettre à la troisième couche d'interface tissulaire (420, 915) de se séparer de la deuxième couche d'interface tissulaire (210, 310, 410, 910, 1010, 1110) lorsqu'une force supérieure à la puissance de liaison est appliquée pour tirer la troisième couche d'interface tissulaire (420, 915) à l'écart de la deuxième couche d'interface tissulaire (210, 310, 410, 910, 1010, 1110).

6. Interface tissulaire selon la revendication 5, dans laquelle la seconde couche de liaison (425, 925) comprend un adhésif.

7. Interface tissulaire selon la revendication 6, dans laquelle la force de liaison de la seconde couche de liaison (425, 925) est comprise entre 20 N par 20 mm² et 30 N par 20 mm² lorsqu'elle est durcie.

8. Interface tissulaire selon la revendication 5, dans laquelle la puissance de liaison de la première couche de liaison (415, 915) et de la seconde couche de liaison (425, 925) sont des valeurs différentes.

9. Interface tissulaire selon la revendication 5, dans laquelle chacune de la première couche d'interface tissulaire (405, 905), de la deuxième couche d'interface tissulaire (410, 910), et de la troisième couche d'interface tissulaire (420, 915) comprend une feuille présentant une épaisseur inférieure à 10,0 mm.
